(19) **Europäisches Patentamt**
**European Patent Office**
**Office européen des brevets**

(11) **EP 2 868 634 B1**

(12) **EUROPÄISCHE PATENTSCHRIFT**

(45) Veröffentlichungstag und Bekanntmachung des Hinweises auf die Patenterteilung:
**07.07.2021   Patentblatt 2021/27**

(51) Int Cl.:
***C03C 4/00*** *(2006.01)*

(21) Anmeldenummer: **13191689.2**

(22) Anmeldetag: **05.11.2013**

(54) **Lithiumdisilikat-Apatit-Glaskeramik mit Übergangsmetalloxid**

Lithium disilicate apatite glass ceramic with transition metal oxide

Vitrocéramique à l'apatite et au lithium-silice avec oxyde de métal de transition

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.05.2015   Patentblatt 2015/19**

(73) Patentinhaber: **Ivoclar Vivadent AG**
**9494 Schaan (LI)**

(72) Erfinder:
- **Rampf, Markus**
  **8853 Lachen (CH)**
- **Ritzberger, Christian**
  **9472 Grabs (CH)**
- **Schweiger, Marcel**
  **7000 Chur (CH)**
- **Höland, Wolfram**
  **9494 Schaan (LI)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
EP-A1- 1 505 041     WO-A1-2013/164256
WO-A2-2013/053864    US-B1- 6 455 451

- **MARTIN PALOU: "Mechanism and kinetics of glass-ceramics formation in the Li2O-SiO2-CaO-P2O5-CaF2 system", CENTRAL EUROPEAN JOURNAL OF CHEMISTRY, CENTRAL EUROPEAN SCIENCE JOURNALS, PL; US; NL; DE, Bd. 7, Nr. 2, 1. Januar 2009 (2009-01-01), Seiten 228-233, XP002682499, ISSN: 1644-3624**
- **None**

## Beschreibung

Die Erfindung betrifft Lithiumdisilikat-Apatit-Glaskeramik, die Übergangsmetalloxid enthält und sich insbesondere zum Einsatz in der Zahnheilkunde, bevorzugt zur Herstellung von dentalen Restaurationen, eignet sowie Vorstufen zu deren Herstellung.

Glaskeramiken mit einer Lithiumdisilikat- und einer Apatit-Kristallphase sind aus dem Stand der Technik bekannt.

M. Palou et al. berichten in Cent. Eur. J. Chem, 7(2), 228-233 (2009) über die Kristallisation von einer Mischung aus reinem Lithiumdisilikat-Glas und Fluorapatit-Glas. Die dabei erzeugte Glaskeramik hat einen hohen Gehalt von 14 Gew.-% $P_2O_5$ und zeigt Bioaktivität beim in-vitro Test in simulierter Körperflüssigkeit.

S.C. Mojumdar et al. beschreiben in Journal of Thermal Analysis and Calorimetry 78(1), 73-82 (2004) Untersuchungen zur Kristallisation von Gläsern aus dem System $Li_2O$-CaO-$CaF_2$-$P_2O_5$-$SiO_2$ mit unterschiedlichen Gehalten an $P_2O_5$. Nach Kristallisation eines Glases mit einem Gehalt von 15 Gew.-% $P_2O_5$ wurde neben einer Lithiumdisilikat-Kristallphase auch Fluorapatit mittels Röntgenbeugung nachgewiesen.

Bei den aus dem Stand der Technik bekannten Lithiumsilikat-Glaskeramiken mit Apatit-Kristallphase handelt es sich jedoch um bioaktive Produkte und nicht um chemisch beständige Materialien, die für die restaurative Zahnmedizin geeignet sind. Bioaktive Produkte bilden in Körperflüssigkeiten oder simulierten Körperflüssigkeiten Apatitkristalle auf der Oberfläche aus, um z.B. im Falle eines endoprothetischen Implantats einen festen Verbund mit dem Knochen zu ergeben.

Die bekannten Glaskeramiken leiden daher an dem schwerwiegenden Nachteil, dass sie nicht über die chemische Beständigkeit verfügen, die für ein Dentalmaterial erforderlich ist, welches in Kontakt mit verschiedensten Fluiden in der Mundhöhle kommt.

Die Dokumente WO2013/164256 A1, US 6 455 451 B1, EP 1 505 041 A1 und der Artikel Central European Journal of Chemistry, 2009, 7(2), 228-233 beschreiben Glaskeramiken.

Der Erfindung liegt daher die Aufgabe zugrunde, eine Lithiumdisilikat-Apatit-Glaskeramik zur Verfügung zu stellen, die über eine sehr gute chemische Beständigkeit verfügt und damit als restauratives Dentalmaterial verwendet werden kann. Die Glaskeramik soll darüber hinaus in einfacher Weise zu dentalen Restaurationen verarbeitbar sein und die aus ihr hergestellten Restaurationen sollen neben einer sehr guten chemischen Beständigkeit auch über sehr gute mechanische und optische Eigenschaften verfügen.

Diese Aufgabe wird durch die Lithiumdisilikat-Apatit-Glaskeramik nach den Ansprüchen 1 bis 15 und 17 gelöst. Gegenstand der Erfindung sind ebenfalls die Lithiummetasilikat-Glaskeramik nach Anspruch 16 oder 17, das Verfahren nach Ansprüchen 18 und 19 sowie die Verwendung nach Ansprüchen 20 und 21.

Die erfindungsgemäße Lithiumdisilikat-Apatit-Glaskeramik zeichnet sich dadurch aus, dass sie Lithiumdisilikat als Hauptkristallphase und Apatit als weitere Kristallphase enthält und sie zweiwertiges Oxid ausgewählt aus der Gruppe aus CaO, SrO und Mischungen dieser und Übergangsmetalloxid ausgewählt aus der Gruppe aus Oxiden der Übergangsmetalle mit der Ordnungszahl 39 bis 79 und Mischungen dieser enthält, wobei das Molverhältnis von zweiwertigem Oxid zu Übergangsmetalloxid im Bereich von 1,0 bis 20,0, insbesondere 1,0 bis 17,0 und bevorzugt 1,5 bis 16,5 ist.

Mit dem Begriff "Hauptkristallphase" wird die Kristallphase bezeichnet, die gegenüber anderen Kristallphasen den höchsten Volumenanteil hat.

Überraschenderweise zeichnet sich die erfindungsgemäße Lithiumdisilikat-Apatit-Glaskeramik durch eine sehr hohe chemische Beständigkeit aus. Zur Bestimmung der chemischen Beständigkeit wurde die Glaskeramik gemäß ISO-Norm 6872 (2008) geprüft, indem der Masseverlust bei Lagerung in wässriger Essigsäure bestimmt wurde. Die erfindungsgemäße Lithiumdisilikat-Apatit-Glaskeramik zeigte dabei insbesondere einen Masseverlust von weniger als 100 $\mu g/cm^2$, bevorzugt weniger als 90 und besonders bevorzugt weniger als 80 $\mu g/cm^2$ und ganz besonders bevorzugt weniger als 50 $\mu g/cm^2$.

Weiter ist es innerhalb des angegebenen Bereichs für das Molverhältnis von zweiwertigem Oxid zu Übergangsmetalloxid überraschenderweise möglich, die Transluzenz der erfindungsgemäßen Lithiumdisilikat-Apatit-Glaskeramik gezielt einzustellen. Während der Einbau des Übergangsmetalloxids zu einer Steigerung der Transluzenz führt, verringert die Kristallisation von Apatit die Transluzenz der erfindungsgemäßen Glaskeramik. Durch diese beiden einander gegenläufigen Effekte kann daher die Transluzenz in gewünschter Weise eingestellt werden. Dies ist gerade für Dentalmaterialien ein außerordentlicher Vorteil, da je nach deren Verwendungsweise ein unterschiedliches Maß an Lichtdurchlässigkeit gewünscht ist. Bei den in der Glaskeramik vorhandenen Übergangsmetalloxiden handelt es sich um sterisch verhältnismäßig große Baugruppen, so dass ihr Einbau zu einer signifikanten Veränderung des Gefüges führen sollte. Es ist daher überraschend, dass trotz des Einbaus von Übergangsmetalloxid die gleichzeitige Kristallisation von sowohl Lithiumdisilikat als auch Apatit gelingt.

Die erfindungsgemäße Lithiumdisilikat-Apatit-Glaskeramik enthält vorzugsweise 52,0 bis 75,0, insbesondere 54,0 bis 73,0 Gew.-% $SiO_2$.

Auch ist es bevorzugt, dass die Lithiumdisilikat-Apatit-Glaskeramik 10,0 bis 20,0, insbesondere 12,0 bis 20,0

Gew.-% Li$_2$O enthält.

Das molare Verhältnis von SiO$_2$ zu Li$_2$O in der Glaskeramik liegt insbesondere im Bereich von 1,5 bis 3,0.

Weiter ist eine Lithiumdisilikat-Apatit-Glaskeramik bevorzugt, die 4,0 bis 8,0 Gew.-% P$_2$O$_5$ enthält.

Die erfindungsgemäße Glaskeramik enthält insbesondere 2,0 bis 9,0, bevorzugt 3,0 bis 8,0 Gew.-% des zweiwertigen Oxids oder Mischungen davon.

In einer weiteren bevorzugten Ausführungsform enthält die erfindungsgemäße Glaskeramik 2,5 bis 8,5, insbesondere 3,0 bis 8,0 Gew.-% CaO und/oder 1,0 bis 6,5, insbesondere 1,0 bis 6,0 Gew.-% SrO.

Auch ist eine Lithiumdisilikat-Apatit-Glaskeramik bevorzugt, die 0,1 bis 1,5, insbesondere 0,3 bis 1,0 Gew.-% F enthält.

Durch den Einsatz von Fluor ist die Bildung von Fluorapatit möglich. Es ist besonders bevorzugt, dass die erfindungsgemäße Glaskeramik Fluorapatit als Apatit enthält. Der Fluorapatit liegt dabei je nach Kation insbesondere als Ca-Fluorapatit, Sr-Fluorapatit oder gemischter Ca/Sr-Fluorapatit vor.

Es ist eine erfindungsgemäße Glaskeramik bevorzugt, bei der die Apatit-Kristallphase 0,5 bis 10, insbesondere 1 bis 10 und bevorzugt 2 bis 8 Gew.-% der Glaskeramik ausmacht und/oder die Apatitkristalle eine mittlere Größe von 5 bis 500, insbesondere 10 bis 300 und bevorzugt 20 bis 200 nm aufweisen.

Die mittlere Größe der Kristalle wurde aus den Röntgenbeugungsdiagrammen unter Benutzung der Scherrer-Gleichung als L berechnet:

$$\Delta(2\theta) = \frac{K\lambda}{L\cos\theta}$$

K: Scherrer-Formfaktor

$\lambda$: Wellenlänge

$\theta$: Beugungswinkel

L: Ausdehnung Kristallit senkrecht zur Netzebene (mittlere Kristallitgrösse)

Dabei wurde als Referenzmuster für die Apatitkristalle die JCPDS 01-074-4390 Kartei herangezogen.

In einer bevorzugten Ausführungsform enthält die Glaskeramik auch 0 bis 4,0, insbesondere 1,0 bis 4,0 und bevorzugt 1,5 bis 4,0 Gew.-% Al$_2$O$_3$.

Die erfindungsgemäße Glaskeramik enthält üblicherweise 0,5 bis 8,5, insbesondere 1,0 bis 8,0 und bevorzugt 2,0 bis 7,5 Gew.-% des Übergangsmetalloxids oder Mischungen davon.

Das in der Glaskeramik vorhandene Übergangsmetalloxid ist bevorzugt aus der Gruppe aus La$_2$O$_3$, Y$_2$O$_3$, Er$_2$O$_3$, ZrO$_2$, CeO$_2$, Tb$_4$O$_7$, V$_2$O$_5$, Ta$_2$O$_5$, Nb$_2$O$_5$ und Mischungen davon ausgewählt.

Weiter ist eine erfindungsgemäße Glaskeramik bevorzugt, bei der Übergangsmetalloxid

entsprechend der Formel Me$_2$O$_3$ in einer Menge von 0 bis 5,0, insbesondere 2,5 bis 4,0 Gew.-%,

entsprechend der Formel MeO$_2$ in einer Menge von 0 bis 6,5, insbesondere 1,0 bis 6,0 Gew.-%,

entsprechend der Formel Me$_4$O$_7$ in einer Menge von 0 bis 1,0, insbesondere 0,4 bis 1,0 Gew.-% und/oder

entsprechend der Formel Me$_2$O$_5$ in einer Menge von 0 bis 5,0, insbesondere 0,1 bis 4,0 Gew.-%,

vorhanden ist.

Dabei steht "Me" in den angegebenen Formeln für das jeweilige Übergangsmetall der Ordnungszahl 39 bis 79.

Die erfindungsgemäße Lithiumdisilikat-Apatit-Glaskeramik enthält üblicherweise einwertiges Oxid ausgewählt aus der Gruppe aus Na$_2$O, K$_2$O, Rb$_2$O, Cs$_2$O und Mischungen dieser in einer Menge von 0 bis 12,0, insbesondere 2,0 bis 12,0 und bevorzugt 3,0 bis 11,5 Gew.-%. enthält.

Weiter ist eine Lithiumdisilikat-Apatit-Glaskeramik bevorzugt, die mindestens eine und insbesondere alle folgenden Komponenten enthält:

| Komponente | Gew.-% |
|---|---|
| SiO$_2$ | 52,0 bis 75,0 |
| Li$_2$O | 10,0 bis 20,0 |

(fortgesetzt)

| Komponente | Gew.-% |
|---|---|
| $P_2O_5$ | 4,0 bis 8,0, |
| zweiwertiges Oxid | 2,0 bis 9,0 |
| F | 0,1 bis 1,5 |
| $Al_2O_3$ | 0 bis 4,0, |
| Übergangsmetalloxid | 0,5 bis 8,5 |
| einwertiges Oxid | 0 bis 12,0. |

Die erfindungsgemäße Lithiumdisilikat-Apatit-Glaskeramik kann darüber hinaus noch Zusatzkomponenten enthalten, die insbesondere ausgewählt sind aus Färbemitteln und Fluoreszenzmitteln. Beispiel für Färbemittel und Fluoreszenzmittel sind Oxide von d- und f-Elementen.

In einer weiteren bevorzugten Ausführungsform enthält die erfindungsgemäße Lithiumdisilikat-Apatit-Glaskeramik mehr als 10 Vol.-%, bevorzugt mehr als 20 Vol.-% und besonders bevorzugt mehr als 30 Vol.-% an Lithiumdisilikat-Kristallen, bezogen auf die gesamte Glaskeramik.

Die erfindungsgemäße Glaskeramik mit Lithiumdisilikat als Hauptkristallphase zeichnet sich durch besonders gute mechanische Eigenschaften aus, und sie kann z.B. durch Wärmebehandlung eines entsprechenden Ausgangsglases oder eines entsprechenden Ausgangsglases mit Keimen oder einer entsprechenden Lithiummetasilikat-Glaskeramik gebildet werden.

Es hat sich weiter gezeigt, dass die erfindungsgemäße Lithiumdisilikat-Apatit-Glaskeramik über eine ausgezeichnet chemische Beständigkeit verfügt und zudem sehr gute mechanische und optische Eigenschaften aufweist. Weiter kann bei ihr die Transluzenz durch das Übergangsmetalloxid sowie die Apatit-Kristallphase gezielt eingestellt werden, indem einerseits die Transluzenz steigernde Wirkung des Übergangsmetalloxids und andererseits die Transluzenz verringernde Wirkung des Apatits ausgenutzt wird. Auch kann bei ihr der lineare thermische Ausdehnungskoeffizient in einem breiten Bereich eingestellt werden.

Sie ist damit den bekannten Lithiumdisilikat-Apatit-Glaskeramiken überlegen. Die Kombination ihrer Eigenschaften erlaubt es sogar, sie als Dentalmaterial und insbesondere als Material zur Herstellung von Dentalrestaurationen einzusetzen.

Die erfindungsgemäße Lithiumdisilikat-Apatit-Glaskeramik hat insbesondere eine Bruchzähigkeit, gemessen als $K_{IC}$ Wert, von mindestens etwa 1,5 MPa•m$^{0.5}$ und insbesondere mindestens etwa 1,8 MPa•m$^{0.5}$. Dieser Wert wurde mit dem Vicker's-Verfahren bestimmt und mittels Niihara-Gleichung berechnet. Weiter hat sie eine hohe biaxiale Bruchfestigkeit von insbesondere mindestens etwa 200 und bevorzugt mindestens etwa 300 MPa. Die biaxiale Bruchfestigkeit wurde gemäß ISO 6872 (2008) bestimmt.

Ebenfalls werden verschiedene Vorstufen mit entsprechender Zusammensetzung offenbart, aus denen die erfindungsgemäße Lithiumdisilikat-Apatit-Glaskeramik durch Wärmebehandlung hergestellt werden kann. Diese Vorstufen sind ein entsprechendes Ausgangsglas, ein entsprechendes Ausgangsglas mit Keimen und eine entsprechende Lithiummetasilikat-Glaskeramik.

Offenbart wird daher ebenfalls ein Ausgangsglas, das die Komponenten der erfindungsgemäßen Lithiumdisilikat-Apatit-Glaskeramik enthält.

Das Ausgangsglas enthält daher zweiwertiges Oxid ausgewählt aus der Gruppe aus CaO, SrO und Mischungen dieser und Übergangsmetalloxid ausgewählt aus der Gruppe aus Oxiden der Übergangsmetalle mit der Ordnungszahl 39 bis 79 und Mischungen dieser, wobei das Molverhältnis von zweiwertigem Oxid zu Übergangsmetalloxid im Bereich von 1,0 bis 20,0, insbesondere 1,0 bis 17,0 und bevorzugt 1,5 bis 16,5 ist.

Das Ausgangsglas enthält darüber hinaus auch insbesondere geeignete Mengen an weiteren Komponenten, die zur Ausbildung der erfindungsgemäßen Glaskeramik mit Lithiumdisilikat als Hauptkristallphase und Apatit als weiterer Kristallphase erforderlich sind. Bevorzugt enthält es $SiO_2$ und $Li_2O$ in Mengen, die die Ausbildung von Lithiumdisilikat ermöglichen. Weiter kann das Ausgangsglas auch noch andere Komponenten enthalten, wie sie oben für die erfindungsgemäße Lithiumdisilikat-Apatit-Glaskeramik angegeben sind. Es sind alle solche Ausführungsformen für die Komponenten des Ausgangsglases bevorzugt, die auch für die Komponenten der erfindungsgemäßen Lithiumdisilikat-Apatit-Glaskeramik als bevorzugt angegeben sind.

Ebenfalls offenbart wird ein solches Ausgangsglas, das Keime für die Kristallisation von Lithiummetasilikat, Lithiumdisilikat und/oder Apatit enthält.

Weiter betrifft die Erfindung eine Lithiummetasilikat-Glaskeramik, die Lithiummetasilikat insbesondere als Hauptkristallphase und die Komponenten der erfindungsgemäßen Lithiumdisilikat-Apatit-Glaskeramik enthält.

Die erfindungsgemäße Lithiummetasilikat-Glaskeramik enthält daher zweiwertiges Oxid ausgewählt aus der Gruppe aus CaO, SrO und Mischungen dieser und Übergangsmetalloxid ausgewählt aus der Gruppe aus Oxiden der Übergangsmetalle mit der Ordnungszahl 39 bis 79 und Mischungen dieser, wobei das Molverhältnis von zweiwertigem Oxid zu Übergangsmetalloxid im Bereich von 1,0 bis 20,0, insbesondere 1,0 bis 17,0 und bevorzugt 1,5 bis 16,5 ist.

Die erfindungsgemäße Lithiummetasilikat-Glaskeramik enthält darüber hinaus auch insbesondere geeignete Mengen an weiteren Komponenten, die zur Ausbildung der erfindungsgemäßen Glaskeramik mit Lithiumdisilikat als Hauptkristallphase und Apatit als weiterer Kristallphase erforderlich sind. Weiter kann die Lithiummetasilikat-Glaskeramik auch noch andere Komponenten enthalten, wie sie oben für die erfindungsgemäße Lithiumdisilikat-Apatit-Glaskeramik angegeben sind. Es sind alle solche Ausführungsformen für die Komponenten der Lithiummetasilikat-Glaskeramik bevorzugt, die auch für die Komponenten der erfindungsgemäßen Lithiumdisilikat-Apatit-Glaskeramik als bevorzugt angegeben sind.

In einer weiteren Ausführungsform enthält die erfindungsgemäße Lithiummetasilikat-Glaskeramik auch Apatit und/oder Lithiumdisilikat als weitere Kristallphase(n).

Durch Wärmebehandlung des Ausgangsglases können zunächst die weiteren Vorstufen Ausgangsglas mit Keimen und Lithiummetasilikat-Glaskeramik erzeugt werden. Durch Wärmebehandlung von einer dieser beiden weiteren Vorstufen kann dann die erfindungsgemäße Lithiumdisilikat-Apatit-Glaskeramik erzeugt werden. Es ist bevorzugt, die erfindungsgemäße Lithiumdisilikat-Apatit-Glaskeramik direkt durch Wärmebehandlung des Ausgangsglases mit Keimen zu bilden.

Es ist bevorzugt, das Ausgangsglas einer Wärmebehandlung bei einer Temperatur von 450 bis 600°C, insbesondere 450 bis 550°C, für eine Dauer von 5 bis 120 min, insbesondere 10 bis 60 min, zu unterwerfen, um das Ausgangsglas mit Keimen für die Kristallisation von Lithiummetasilikat, Lithiumdisilikat und/oder Apatit zu erzeugen.

Es ist weiter bevorzugt, das Ausgangsglas mit Keimen einer Wärmebehandlung bei einer Temperatur von mehr als 600°C für eine Dauer von 5 bis 120 min, insbesondere 10 bis 60 min, zu unterwerfen, um die Lithiummetasilikat-Glaskeramik oder die Lithiumdisilikat-Apatit-Glaskeramik herzustellen. Zur Herstellung der Lithiumdisilikat-Apatit-Glaskeramik erfolgt die Wärmebehandlung des Ausgangsglases mit Keimen besonders bevorzugt bei 700 bis 1000°C, insbesondere 750 bis 950°C, für eine Dauer von 5 bis 120 min, insbesondere 10 bis 60 min.

Die Erfindung betrifft ebenfalls ein Verfahren zur Herstellung der erfindungsgemäßen Lithiumdisilikat-Apatit-Glaskeramik, bei dem das Ausgangsglas, das Ausgangsglas mit Keimen oder die Lithiummetasilikat-Glaskeramik mindestens einer Wärmebehandlung im Bereich von 450 bis 1000°C unterzogen wird.

Die im erfindungsgemäßen Verfahren durchgeführte mindestens eine Wärmebehandlung kann auch im Rahmen eines Heißpressens oder Aufsinterns des Ausgangsglases, des Ausgangsglases mit Keimen oder der erfindungsgemäßen Lithiummetasilikat-Glaskeramik erfolgen.

In einer bevorzugten Ausführungsform umfasst das erfindungsgemäße Verfahren

(a) die Wärmebehandlung des Ausgangsglases bei einer Temperatur von 450 bis 600°C, um das Ausgangsglas mit Keimen zu bilden, und

(b) die Wärmebehandlung des Ausgangsglases mit Keimen bei einer Temperatur von 700 bis 1000°C, um die Lithiumdisilikat-Apatit-Glaskeramik zu bilden.

Die Dauer der bei (a) und (b) durchgeführten Wärmebehandlungen beträgt insbesondere 5 bis 120 min und bevorzugt 10 bis 60 min. Zur Herstellung des Ausgangsglases wird insbesondere so vorgegangen, dass eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen gegossen werden, um Rohlinge des Ausgangsglases, sogenannte Massivglasrohlinge oder monolithische Rohlinge, zu erzeugen.

Es ist ebenfalls möglich, die Schmelze erneut in Wasser zu geben, um ein Granulat herzustellen. Dieses Granulat kann nach Mahlen und gegebenenfalls Zugabe weiterer Komponenten, wie Färbe- und Fluoreszenzmitteln, zu einem Rohling, einem sogenannten Pulverpressling, gepresst werden.

Schließlich kann das Ausgangsglas nach Granulierung auch zu einem Pulver verarbeitet werden.

Anschließend wird das Ausgangsglas, z.B. in Form eines Massivglasrohlings, eines Pulverpresslings oder in Form eines Pulvers, mindestens einer Wärmebehandlung unterzogen. Es ist bevorzugt, dass zunächst eine erste Wärmebehandlung durchgeführt wird, um ein Ausgangsglas mit Keimen herzustellen, welche zur Bildung von Lithiummetasilikat-, Lithiumdisilikat- und/oder Apatit-Kristallen geeignet sind. Das Glas mit Keimen wird dann üblicherweise mindestens einer weiteren Temperaturbehandlung bei einer höheren Temperatur unterworfen, um Kristallisation von Lithiummetasilikat, Lithiumdisilikat und/oder Apatit zu bewirken.

Zur Kristallisation von Lithiummetasilikat erfolgt die weitere Wärmebehandlung insbesondere bei einer Temperatur von mindestens 600°C. Zur Kristallisation von Lithiumdisilikat erfolgt die weitere Wärmebehandlung insbesondere bei einer Temperatur von mindestens 700°C. Zur Kristallisation von Apatit erfolgt die weitere Wärmebehandlung insbesondere bei einer Temperatur von mindestens 750°C.

Die erfindungsgemäßen Glaskeramiken und die oben beschriebenen Gläser liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden. Sie können aber auch in Form von dentalen Restaurationen, wie Brücken, Inlays, Onlays, Kronen, Veneers, Schalen oder Abutments, vorliegen.

Aus den erfindungsgemäßen Glaskeramiken und den oben beschriebenen Gläsern können dentale Restaurationen, wie Brücken, Inlays, Onlays, Kronen, Veneers, Schalen oder Abutments, hergestellt werden. Die Erfindung betrifft daher auch deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 2 bis 10 bar durchzuführen. Beim Verpressen wird durch viskoses Fließen des eingesetzten Materials die gewünschte Formänderung erreicht. Es können für das Verpressen das Ausgangsglas und insbesondere das Ausgangsglas mit Keimen, die erfindungsgemäße Lithiummetasilikat-Glaskeramik und die erfindungsgemäße Lithiumdisilikat-Apatit-Glaskeramik verwendet werden. Dabei können die Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das oben beschriebene Ausgangsglas, das oben beschriebenen Ausgangsglas mit Keimen, die erfindungsgemäße Lithiummetasilikat-Glaskeramik und die erfindungsgemäße Lithiumdisilikat-Apatit-Glaskeramik verwendet werden. Dabei können die Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die erfindungsgemäße Lithiummetasilikat-Glaskeramik oder die erfindungsgemäße Lithiumdisilikat-Apatit-Glaskeramik verwendet. Die Lithiumdisilikat-Apatit-Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich ist. Nach der maschinellen Bearbeitung eines solchen teilkristallisierten Materials wird dieses regelmäßig einer Wärmebehandlung bei höherer Temperatur und insbesondere 700 bis 1000°C und bevorzugt 750 bis 950°C unterzogen, um eine weitere Kristallisation von Lithiumdisilikat und Apatit hervorzurufen.

Allgemein kann nach der Herstellung der gewünscht geformten dentalen Restauration, z. B. durch Verpressen oder maschinelle Bearbeitung, diese insbesondere noch wärmebehandelt werden, um eingesetzte Vorläufer, wie Ausgangsglas, Ausgangsglas mit Keimen oder Lithiummetasilikat-Glaskeramik in Lithiumdisilikat-Apatit-Glaskeramik umzuwandeln oder die Kristallisation von Lithiumdisilikat und/oder Apatit zu steigern oder die Porosität, z.B. eines eingesetzten porösen Pulverpressling, zu vermindern.

Die erfindungsgemäßen Glaskeramiken und die oben beschriebenen Gläser eignen sich allerdings auch als Beschichtungsmaterial von z.B. Keramiken, wie $ZrO_2$-Keramiken, und Glaskeramiken. Die Erfindung ist daher ebenfalls auf die Verwendung der Gläser oder der erfindungsgemäßen Glaskeramiken zur Beschichtung von insbesondere Keramiken und Glaskeramiken gerichtet. Dabei erweist es sich als sehr günstig, dass die erfindungsgemäßen Glaskeramiken einen linearen thermische Ausdehnungskoeffizienten aufweisen, der in einem breiten Bereich von insbesondere 8,0 bis 13,5 x $10^{-6}K^{-1}$ (gemessen im Bereich von 100 bis 500°C) liegt. Damit steht für unterschiedlichste Anwendungen eine erfindungsgemäße Glaskeramik mit gewünschtem Ausdehnungskoeffizienten zur Verfügung.

Die Erfindung betrifft auch ein Verfahren zur Beschichtung von Keramiken und Glaskeramiken, bei dem erfindungsgemäße Glaskeramiken oder Gläser auf die Keramik oder Glaskeramik aufgebracht und erhöhter Temperatur ausgesetzt werden.

Dies kann insbesondere durch Aufsintern und bevorzugt durch Aufpressen erfolgen. Beim Aufsintern wird die Glaskeramik oder das Glas in üblicher Weise, z.B. als Pulver, auf das zu beschichtende Material, wie Keramik oder Glaskeramik, aufgebracht und anschließend bei erhöhter Temperatur gesintert. Bei dem bevorzugten Aufpressen wird erfindungsgemäße Glaskeramik oder Glas, z.B. in Form von Pulverpresslingen oder monolithischen Rohlingen, bei einer erhöhten Temperatur, von z.B. 700 bis 1200°C, und unter Anwendung von Druck, z.B. 2 bis 10 bar, aufgepresst. Hierzu können insbesondere die in der EP 231 773 beschriebenen Verfahren und der dort offenbarte Pressofen eingesetzt werden. Ein geeigneter Ofen ist z.B. der Programat EP 5000 von Ivoclar Vivadent AG, Liechtenstein.

Es ist bevorzugt, dass nach Abschluss des Beschichtungsvorganges die erfindungsgemäße Glaskeramik mit Lithiumdisilikat als Hauptkristallphase und Apatit als weiterer Kristallphase vorliegt, da eine solche Glaskeramik über besonders gute Eigenschaften verfügt.

Aufgrund der vorstehend geschilderten Eigenschaften der erfindungsgemäßen Glaskeramiken und der oben beschriebenen Gläser eignen sich diese insbesondere zum Einsatz in der Zahnheilkunde. Gegenstand der Erfindung ist daher auch die Verwendung der erfindungsgemäßen Glaskeramiken als Dentalmaterial und insbesondere zur Herstellung dentaler Restaurationen oder als Beschichtungsmaterial für dentale Restaurationen, wie Kronen, Brücken und Abutments.

Die Erfindung wird im Folgenden anhand von sie nichtbeschränkenden Beispielen näher erläutert.

Beispiele

Beispiele 1 bis 20 - Zusammensetzung und Kristallphasen

Es wurden insgesamt 20 Gläser und erfindungsgemäße Glaskeramiken mit der in der unten stehenden Tabelle angegebenen Zusammensetzung über Erschmelzung entsprechender Ausgangsgläser und anschließende Wärmebehandlung zur gesteuerten Keimbildung und Kristallisation hergestellt.

Die $T_g$-Werte der Gläser sowie die angewendeten Wärmebehandlungen zur gesteuerten Keimbildung und gesteuerten Kristallisation sind ebenfalls in der Tabelle angegeben. Dabei bedeuten

| | |
|---|---|
| $T_N$ und $t_N$ | Angewendete Temperatur und Zeit für Keimbildung |
| $T_{K1}$ und $t_{K1}$ | Angewendete Temperatur und Zeit für eine 1. Kristallisation |
| $T_{K2}$ und $t_{K2}$ | Angewendete Temperatur und Zeit für eine 2. Kristallisation |

Dazu wurden zunächst die Ausgangsgläser im 100 bis 200 g Maßstab aus üblichen Rohstoffen bei 1400 bis 1500°C erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1450 bis 1550°C für 1 bis 3 h geschmolzen wurden.

Eine Wärmebehandlung der Ausgangsgläser bei einer Temperatur von 460 bis 540°C führte zur Bildung von Lithiumsilikat-Gläsern mit Keimen.

Diese keimhaltigen Gläser kristallisierten durch mindestens eine weitere Wärmebehandlung zu Glaskeramiken mit Lithiummetasilikat als Hauptkristallphase oder Glaskeramiken mit Lithiumdisilikat als Hauptkristallphase und Apatit als weiterer Kristallphase, wie durch Röntgenbeugungsuntersuchungen festgestellt wurde. Der Apatit lag dabei als Ca-Fluorapatit, Sr-Fluorapatit oder Ca/Sr-Fluorapatit vor.

Bei den Beispielen 1 bis 4, 7, 9, 10 und 15 führte eine erste weitere Wärmebehandlung $T_{K1}$ der keimhaltigen Ausgangsgläser zu Glaskeramiken mit Lithiummetasilikat als Hauptkristallphase und die erhaltenen Lithiummetasilikat-Glaskeramiken wurden durch eine zweite weitere Wärmebehandlung $T_{K2}$ zu Glaskeramiken mit Lithiumdisilikat als Hauptkristallphase und Apatit als weiterer Kristallphase umgewandelt.

Bei den Beispielen 13, 14, 16, 17, 19 und 20 wurden die keimhaltigen Ausgangsgläser durch lediglich eine weitere Wärmebehandlung zu Glaskeramiken mit Lithiumdisilikat als Hauptkristallphase und Apatit als weiterer Kristallphase und im Falle von Beispiel 18 zu einer Glaskeramik mit Lithiummetasilikat als Hauptkristallphase und Apatit als weiterer Kristallphase umgewandelt.

Bei den Beispielen 8, 11 und 12 lagen auch nach der zweiten weiteren Wärmebehandlung Glaskeramiken mit Lithiummetasilikat als Hauptkristallphase vor.

Schließlich zeigen die Beispiele 5 und 6 die Erzeugung von Glaskeramiken mit Lithiumdisilikat als Hauptkristallphase bereits nach der ersten weiteren Wärmebehandlung und deren weitere Kristallisation durch eine zweite weitere Wärmebehandlung.

Die Beispiele zeigen damit insgesamt, wie durch Veränderung der Zusammensetzung der Ausgangsgläser und deren Wärmebehandlung unterschiedliche erfindungsgemäße Glaskeramiken erzeugt werden können.

Die erhaltenen erfindungsgemäßen Lithiumdisilikat-Apatit-Glaskeramiken zeigten eine ausgezeichnete chemische Beständigkeit gemäß ISO-Test 6872 (2008). Der Masseverlust bei Lagerung in wässriger Essigsäure betrug weniger als 100 $\mu g/cm^2$, insbesondere weniger als 50 $\mu g/cm^2$.

Demgegenüber zeigen konventionelle bioaktive Glaskeramiken einen sehr hohen Masseverlust und damit eine sehr geringe chemische Beständigkeit. Sie sind zum Einsatz als restauratives Dentalmaterial nicht geeignet, welches in der Mundhöhle stetig mit Fluiden unterschiedlichster Zusammensetzung in Kontakt kommt.

Die erzeugten Lithiumdisilikat-Apatit-Glaskeramiken hatten auch eine sehr hohe Biaxialfestigkeit $\sigma_B$ von mehr als 390 und insbesondere von bis zu etwa 640 MPa. Diese Festigkeit wurde gemäß Dentalnorm ISO 6872 (2008) an Prüfkörpern bestimmt. Die Prüfkörper wurden durch maschinelle Bearbeitung der nach der 1. Kristallisation ($T_{K1}$) erhaltenen Lithiummetasilikat-Glaskeramik und anschließende 2. Kristallisation ($T_{K2}$) zur jeweiligen Lithiumdisilikat-Apatit-Glaskeramik hergestellt. Zur maschinellen Bearbeitung der Lithiummetasilikat-Glaskeramik wurde eine CEREC®-InLab Maschine (Sirona, Bensheim) verwendet.

Die erzeugten Lithiumdisilikat-Apatit-Glaskeramiken und die als Vorstufe erzeugten Lithiummetasilikat-Glaskeramiken konnten sehr gut maschinell in einem CAD/CAM-Verfahren oder durch Heißpressen in die Form verschiedener Dentalrestaurationen gebracht werden, die bei Bedarf noch mit einer Verblendung versehen wurden. Dabei erwiesen sich die Lithiummetasilikat-Glaskeramiken aufgrund ihrer mechanischen Eigenschaften als besonders geeignet für eine Formgebung durch maschinelle Bearbeitung.

Ebenfalls konnten die Glaskeramiken durch Heißpressen als Beschichtungen auf insbesondere Dentalrestaurationen aufgebracht werden, z.B. um diese in gewünschter Weise zu verblenden.

Schließlich wiesen die Glaskeramiken einen linearen Wärmeausdehnungskoeffizienten (WAK) im breiten Bereich von 8,6 bis 11,1 x $10^{-6}$K$^{-1}$ (gemessen im Bereich von 100 bis 500°C) auf. Gerade Materialien mit einem WAK von weniger als 10 x $10^{-6}$K$^{-1}$ sind für die Verblendung von z.B. ZrO$_2$-Keramiken besonders gut geeignet.

In der folgenden Tabelle steht bei der Angabe der Molverhältnisse "ÜMO" für Übergangsmetalloxide.

"RT-XRD" steht für Röntgenbeugungsuntersuchungen bei Raumtemperatur.

"WAK" steht für linearen thermischen Ausdehnungskoeffizienten.

| Beispiel | 1 | 2 | 3 | 4 | 5 | 6 |
|---|---|---|---|---|---|---|
| | | | | | | |
| SiO$_2$ | 65,3 | 64,1 | 65,3 | 63,3 | 62,1 | 72,7 |
| GeO$_2$ | - | - | - | - | 2,9 | - |
| Li$_2$O | 13,5 | 15,0 | 13,5 | 13,3 | 13,5 | 12,1 |
| P$_2$O$_5$ | 5,7 | 4,5 | 5,7 | 5,2 | 4,3 | 4,3 |
| Al$_2$O$_3$ | 3,2 | 3,2 | 3,2 | 2,4 | 3,6 | 1,9 |
| K$_2$O | 3,7 | 3,7 | 3,7 | - | 3,7 | 3,0 |
| Rb$_2$O | - | - | - | 7,7 | - | - |
| Cs$_2$O | - | - | - | - | - | - |
| CaO | 4,1 | 5,0 | 4,1 | 4,1 | 4,0 | 3,0 |
| SrO | - | - | - | - | 1,4 | - |
| F | 0,5 | 0,5 | 0,5 | 0,4 | 0,5 | 0,5 |
| ZrO$_2$ | - | - | - | - | - | - |
| CeO$_2$ | - | - | - | - | - | - |
| La$_2$O$_3$ | - | - | 4,0 | - | 4,0 | 2,5 |
| Y$_2$O$_3$ | - | - | - | 3,6 | - | - |
| V$_2$O$_5$ | - | - | - | - | - | - |
| Ta$_2$O$_5$ | - | 4,0 | - | - | - | - |
| Nb$_2$O$_5$ | 4,0 | - | - | - | - | - |
| Tb$_4$O$_7$ | - | - | - | - | - | - |
| Er$_2$O$_3$ | - | - | - | - | - | - |
| Σ | 100,0 | 100,0 | 100,0 | 100,0 | 100,0 | 100,0 |
| | | | | | | |
| Molverhältnis (CaO+SrO) zu ÜMO | 4,7 | 10,0 | 6,0 | 5,4 | 7,0 | 7,5 |

| Beispiel | 1 | 2 | 3 | 4 | 5 | 6 |
|---|---|---|---|---|---|---|
| $T_g$ / °C | 467 | 453 | 458 | 469 | 453 | 456 |
| $T_N$ / °C | 520 | 480 | 500 | 490 | 540 | 460 |
| $t_N$ / min. | 20 | 40 | 10 | 40 | 10 | 30 |
| $T_{K1}$ / °C | 670 | 650 | 640 | 620 | 680 | 600 |
| $t_{K1}$ / min. | 10 | 20 | 40 | 30 | 40 | 30 |
| **RT-XRD nach $T_{K1}$** | | | | | | |
| Hauptkristallphase | $Li_2SiO_3$ | $Li_2SiO_3$ | $Li_2SiO_3$ | $Li_2SiO_3$ | $Li_2Si_2O_5$ | $Li_2Si_2O_5$ |
| Weitere Kristallphasen | $Li_2Si_2O_5$, $Li_3PO_4$ | $Li_2Si_2O_5$ | $Li_3PO_4$ | - | $Li_2SiO_3$, $Li_3PO_4$, $Ca_5(PO_4)_3F$ | $Li_2SiO_3$ |
| $T_{K2}$ / °C | 800 | 800 | 760 | 800 | 820 | 810 |
| $t_{K2}$ / min. | 10 | 15 | 60 | 30 | 10 | 20 |
| **RT-XRD nach $T_{K2}$** | | | | | | |
| Hauptkristallphase | $Li_2Si_2O_5$ | $Li_2Si_2O_5$ | $Li_2Si_2O_5$ | $Li_2Si_2O_5$ | $Li_2Si_2O_5$ | $Li_2Si_2O_5$ |
| Weitere Kristallphasen | $Li_3PO_4$, $Ca_5(PO_4)_3F$ | $Li_2SiO_3$, $Li_3PO_4$, $Ca_5(PO_4)_3F$ | $Li_3PO_4$, $Ca_5(PO_4)_3F$ | $Li_3PO_4$, $Ca_5(PO_4)_3F$ | $Li_2SiO_3$, $Li_3PO_4$, $Ca_{9.37}Sr_{0.63}(PO_4)_3F$ | $Li_3PO_4$, $Ca_5(PO_4)_3F$, $SiO_2$ |
| $WAK_{100\text{-}500°C}$ / $10^{-6} \cdot K^{-1}$ | 10,3 | 11,2 | 10,5 | 10,5 | 11,1 | |
| $\sigma_B$ / MPa | 442 | 637 | 385 | 562 | 548 | |

| Beispiel | 7 | 8 | 9 | 10 | 11 | 12 |
|---|---|---|---|---|---|---|
| | | | | | | |
| $SiO_2$ | 65,3 | 54,9 | 61,5 | 65,3 | 61,8 | 59,9 |
| $GeO_2$ | - | - | - | - | - | - |
| $Li_2O$ | 13,5 | 18,2 | 12,4 | 13,5 | 17,1 | 18,7 |
| $P_2O_5$ | 5,7 | 4,8 | 4,3 | 5,7 | 4,4 | 4,4 |
| $Al_2O_3$ | 3,2 | 3,5 | 3,2 | 3,2 | 3,5 | 3,5 |
| $K_2O$ | 3,7 | 4,6 | - | 3,7 | 3,9 | 3,9 |
| $Rb_2O$ | - | - | - | - | | - |
| $Cs_2O$ | - | - | 11,1 | - | | - |
| $CaO$ | 4,1 | - | 4,5 | 4,1 | 4,0 | 4,0 |
| $SrO$ | - | 6,0 | - | - | - | - |
| $F$ | 0,5 | 0,8 | 0,5 | 0,5 | 0,5 | 0,5 |
| $ZrO_2$ | - | - | - | - | - | - |
| $CeO_2$ | - | 1,8 | - | - | 1,7 | 1,9 |
| $La_2O_3$ | - | - | - | - | - | - |
| $Y_2O_3$ | - | 4,8 | - | 4,0 | - | 2,5 |
| $V_2O_5$ | - | - | - | - | 0,1 | 0,1 |
| $Ta_2O_5$ | 4,0 | - | 2,5 | - | - | - |
| $Nb_2O_5$ | - | - | - | - | 2,5 | - |
| $Tb_4O_7$ | - | 0,5 | - | - | 0,4 | 0,4 |
| $Er_2O_3$ | - | 0,1 | - | - | 0,1 | 0,2 |
| Σ | 100,0 | 100,0 | 100,0 | 100,0 | 100,0 | 100,0 |
| | | | | | | |
| Molverhältnis (CaO+SrO) zu ÜMO | 8,4 | 1,8 | 16,3 | 4,7 | 3,5 | 3,3 |
| | | | | | | |

| Beispiel | 7 | 8 | 9 | 10 | 11 | 12 |
|---|---|---|---|---|---|---|
| $T_g$ / °C | 465 | 441 | 466 | 461 | 447 | 443 |
| $T_N$ / °C | 490 | 470 | 500 | 470 | 500 | 520 |
| $t_N$ / min. | 60 | 60 | 10 | 120 | 10 | 10 |
| $T_{K1}$ / °C | 600 | 700 | 630 | 580 | 650 | 620 |
| $t_{K1}$ / min. | 30 | 20 | 30 | 60 | 20 | 30 |
| RT-XRD nach $T_{K1}$ | | | | | | |
| Hauptkristallphase | $Li_2SiO_3$ | $Li_2SiO_3$ | $Li_2SiO_3$ | $Li_2SiO_3$ | $Li_2SiO_3$ | $Li_2SiO_3$ |
| Weitere Kristallphasen | $Li_2Si_2O_5$, $Li_3PO_4$ | - | $Li_2Si_2O_5$ | $Li_3PO_4$ | $Li_3PO_4$ | - |
| $T_{K2}$ / °C | 770 | 840 | 840 | 790 | 800 | 800 |
| $t_{K2}$ / min. | 30 | 10 | 10 | 20 | 30 | 20 |
| RT-XRD nach $T_{K2}$ | | | | | | |
| Hauptkristallphase | $Li_2Si_2O_5$ | $Li_2SiO_3$ | $Li_2Si_2O_5$ | $Li_2Si_2O_5$ | $Li_2SiO_3$ | $Li_2SiO_3$ |
| Weitere Kristallphasen | $Li_3PO_4$, $Ca_5(PO_4)_3F$ | $Li_3PO_4$, $Sr_5(PO_4)_3F$ | $Cs_{0.809}(AlSi_5O_{12})$, $Li_2SiO_3$, $Li_3PO_4$, $Ca_5(PO_4)_3F$ | $Li_3PO_4$, $Ca_5(PO_4)_3F$ | $Li_2Si_2O_5$, $Li_3PO_4$, $Ca_5(PO_4)_3F$ | $Li_3PO_4$, $Ca_5(PO_4)_3F$ |
| $WAK_{100-500°C}$ / $10^{-6} \cdot K^{-1}$ | 10,2 | | | 10,4 | | |
| $\sigma_B$ / MPa | 419 | | | 392 | | |

| Beispiel | 13 | 14 | 15 | 16 | 17 | 18 |
|---|---|---|---|---|---|---|
| | | | | | | |
| $SiO_2$ | 68,5 | 67,3 | 65,3 | 64,0 | 64,8 | 59,2 |
| $GeO_2$ | - | - | - | - | - | - |
| $Li_2O$ | 14,5 | 14,0 | 13,5 | 13,1 | 13,5 | 19,6 |
| $P_2O_5$ | 4,4 | 5,9 | 5,7 | 5,6 | 5,7 | 5,7 |
| $Al_2O_3$ | 3,5 | 3,3 | 3,2 | 3,2 | 3,2 | 3,2 |
| $K_2O$ | - | 3,8 | 3,7 | 3,6 | 3,7 | 3,7 |
| $Rb_2O$ | - | - | - | - | - | - |
| $Cs_2O$ | - | - | - | - | - | - |
| $CaO$ | 4,0 | 4,2 | 4,1 | 8,0 | 4,1 | 4,1 |
| $SrO$ | - | - | - | - | - | - |
| $F$ | 0,5 | 0,5 | 0,5 | 0,5 | 1,0 | 0,5 |
| $ZrO_2$ | - | 1,0 | 4,0 | 2,0 | 4,0 | 4,0 |
| $CeO_2$ | 1,6 | - | | - | - | - |
| $La_2O_3$ | - | - | - | - | - | - |
| $Y_2O_3$ | 2,5 | - | - | - | - | - |
| $V_2O_5$ | - | - | - | - | - | - |
| $Ta_2O_5$ | 0,5 | - | - | - | - | - |
| $Nb_2O_5$ | - | - | - | - | - | - |
| $Tb_4O_7$ | - | - | - | - | - | - |
| $Er_2O_3$ | - | - | - | - | - | - |
| Σ | 100,0 | 100,0 | 100,0 | 100,0 | 100,0 | 100,0 |
| | | | | | | |
| Molverhältnis (CaO+SrO) zu ÜMO | 3,5 | 8,4 | 2,3 | 8,8 | 2,3 | 2,3 |
| | | | | | | |

| Beispiel | 13 | 14 | 15 | 16 | 17 | 18 |
|---|---|---|---|---|---|---|
| | | | | | | |
| $T_g$ / °C | 443 | 457 | 471 | 463 | 464 | 442 |
| | | | | | | |
| $T_N$ / °C | 480 | 480 | 490 | 490 | 480 | 460 |
| $t_N$ / min. | 10 | 10 | 10 | 10 | 10 | 10 |
| | | | | | | |
| $T_{K1}$ / °C | 890 | - | 650 | - | - | - |
| $t_{K1}$ / min. | 10 | - | 40 | - | - | - |
| | | | | | | |
| RT-XRD nach $T_{K1}$ | | | | | | |
| Hauptkristallphase | $Li_2Si_2O_5$ | | $Li_2SiO_3$ | | | |
| Weitere Kristallphasen | $Li_2O \cdot Al_2O_3 \cdot 7.5SiO_2$, $Li_3PO_4$, $Ca_5(PO_4)_3F$, $SiO_2$ | | $Li_3PO_4$ | | | |
| | | | | | | |
| $T_{K2}$ / °C | - | 790 | 780 | 810 | 780 | 820 |
| $t_{K2}$ / min. | - | 60 | 60 | 60 | 60 | 60 |
| | | | | | | |
| RT-XRD nach $T_{K2}$ | | | | | | |
| Hauptkristallphase | - | $Li_2Si_2O_5$ | $Li_2Si_2O_5$ | $Li_2Si_2O_5$ | $Li_2Si_2O_5$ | $Li_2SiO_3$ |
| Weitere Kristallphasen | - | $Li_3PO_4$, $Ca_5(PO_4)_3F$ | $Li_3PO_4$, $Ca_5(PO_4)_3F$ | $Li_2SiO_3$, $Li_3PO_4$, $Ca_5(PO_4)_3F$ | $Li_3PO_4$, $Ca_5(PO_4)_3F$ | $Li_2Si_2O_5$, $Li_3PO_4$, $Ca_5(PO_4)_3F$ |
| | | | | | | |
| $WAK_{100-500°C}$ / $10^{-6} \cdot K^{-1}$ | | | 8.6 | | | |
| | | | | | | |
| $\sigma_B$ / MPa | | | | | | |

| Beispiel | 19 | 20 |
|---|---|---|
| | | |
| $SiO_2$ | 63,4 | 66,2 |
| $GeO_2$ | - | - |
| $Li_2O$ | 13,4 | 13,6 |
| $P_2O_5$ | 8,0 | 5,8 |
| $Al_2O_3$ | 3,1 | - |
| $K_2O$ | 3,6 | 3,7 |
| $Rb_2O$ | - | - |
| $Cs_2O$ | - | - |
| $CaO$ | 4,0 | 4,2 |
| $SrO$ | - | - |
| $F$ | 0,5 | 0,5 |
| $ZrO_2$ | 4,0 | 6,0 |
| $CeO_2$ | - | - |
| $La_2O_3$ | - | - |
| $Y_2O_3$ | - | - |
| $V_2O_5$ | - | - |
| $Ta_2O_5$ | - | - |
| $Nb_2O_5$ | - | - |
| $Tb_4O_7$ | - | - |
| $Er_2O_3$ | - | - |
| $\Sigma$ | 100,0 | 100,0 |
| | | |
| Molverhältnis (CaO+SrO) zu ÜMO | 2,1 | 1,6 |
| | | |

| Beispiel | 19 | 20 |
|---|---|---|
| | | |
| $T_g$ / °C | 484 | 473 |
| | | |
| $T_N$ / °C | 500 | 490 |
| $t_N$ / min. | 10 | 10 |
| | | |
| $T_{K1}$ / °C | - | - |
| $t_{K1}$ / min. | - | - |
| | | |
| RT-XRD nach $T_{K1}$ | | |
| Hauptkristallphase | | |
| Weitere Kristallphasen | | |
| | | |
| $T_{K2}$ / °C | 800 | 830 |
| $t_{K2}$ / min. | 40 | 60 |
| | | |
| RT-XRD nach $T_{K2}$ | | |
| Hauptkristallphase | $Li_2Si_2O_5$ | $Li_2Si_2O_5$ |
| Weitere Kristallphasen | $Li_3PO_4$, $Ca_5(PO_4)_3F$ | $Li_3PO_4$, $Ca_5(PO_4)_3F$, $KLi_3Zr_2Si_{12}O_{30}$, $SiO_2$ |
| | | |
| $WAK_{100-500°C}$ / $10^{-6} \cdot K^{-1}$ | | |
| | | |
| $\sigma_B$ / MPa | | |

**Patentansprüche**

1. Lithiumdisilikat-Apatit-Glaskeramik, die Lithiumdisilikat als Hauptkristallphase und Apatit als weitere Kristallphase enthält und die zweiwertiges Oxid ausgewählt aus der Gruppe aus CaO, SrO und Mischungen dieser und Übergangsmetalloxid ausgewählt aus der Gruppe aus Oxiden der Übergangsmetalle mit der Ordnungszahl 39 bis 79 und Mischungen dieser enthält, wobei das Molverhältnis von zweiwertigem Oxid zu Übergangsmetalloxid im Bereich von 1,0 bis 20,0, insbesondere 1,0 bis 17,0 und bevorzugt 1,5 bis 16,5 ist.

2. Glaskeramik nach Anspruch 1, die 52,0 bis 75,0, insbesondere 54,0 bis 73,0 Gew.-% $SiO_2$ enthält.

3. Glaskeramik nach Anspruch 1 oder 2, die 10,0 bis 20,0, insbesondere 12,0 bis 20,0 Gew.-% $Li_2O$ enthält.

4. Glaskeramik nach einem der Ansprüche 1 bis 3, die 4,0 bis 8,0 Gew.-% $P_2O_5$ enthält.

5. Glaskeramik nach einem der Ansprüche 1 bis 4, die 2,0 bis 9,0, insbesondere 3,0 bis 8,0 Gew.-% zweiwertiges Oxid enthält.

6. Glaskeramik nach einem der Ansprüche 1 bis 5, die 2,5 bis 8,5, insbesondere 3,0 bis 8,0 Gew.-% CaO enthält.

7. Glaskeramik nach einem der Ansprüche 1 bis 6, die 1,0 bis 6,5, insbesondere 1,0 bis 6,0 Gew.-% SrO enthält

8. Glaskeramik nach einem der Ansprüche 1 bis 7, die 0,1 bis 1,5, insbesondere 0,3 bis 1,0 Gew.-% F enthält.

9. Glaskeramik nach einem der Ansprüche 1 bis 8, die 0 bis 4,0, insbesondere 1,0 bis 4,0 und bevorzugt 1,5 bis 4,0 Gew.-% $Al_2O_3$ enthält.

10. Glaskeramik nach einem der Ansprüche 1 bis 9, die 0,5 bis 8,5, insbesondere 1,0 bis 8,0 und bevorzugt 2,0 bis 7,5 Gew.-% Übergangsmetalloxid enthält.

11. Glaskeramik nach einem der Ansprüche 1 bis 10, bei der das Übergangsmetalloxid ausgewählt ist aus der Gruppe aus $La_2O_3$, $Y_2O_3$, $Er_2O_3$, $ZrO_2$, $CeO_2$, $Tb_4O_7$, $V_2O_5$, $Ta_2O_5$, $Nb_2O_5$ und Mischungen davon.

12. Glaskeramik nach einem der Ansprüche 1 bis 11, bei der Übergangsmetalloxid entsprechend der Formel $Me_2O_3$ in einer Menge von 0 bis 5,0, insbesondere 2,5 bis 4,0 Gew.-%, entsprechend der Formel $MeO_2$ in einer Menge von 0 bis 6,5, insbesondere 1,0 bis 6,0 Gew.-%, entsprechend der Formel $Me_4O_7$ in einer Menge von 0 bis 1,0, insbesondere 0,4 bis 1,0 Gew.-% und/oder entsprechend der Formel $Me_2O_5$ in einer Menge von 0 bis 5,0, insbesondere 0,1 bis 4,0 Gew.-%, vorhanden ist.

13. Glaskeramik nach einem der Ansprüche 1 bis 12, die einwertiges Oxid ausgewählt aus der Gruppe aus $Na_2O$, $K_2O$, $Rb_2O$, $Cs_2O$ und Mischungen dieser in einer Menge von 0 bis 12,0, insbesondere 2,0 bis 12,0 und bevorzugt 3,0 bis 11,5 Gew.-% enthält.

14. Glaskeramik nach einem der Ansprüche 1 bis 13, die Fluorapatit als Apatit enthält.

15. Glaskeramik nach einem der Ansprüche 1 bis 14, bei der die Apatit-Kristallphase 0,5 bis 10, insbesondere 1 bis 10 und bevorzugt 2 bis 8 Gew.-% der Glaskeramik ausmacht und/oder die Apatitkristalle eine mittlere Größe von 5 bis 500, insbesondere 10 bis 300 und bevorzugt 20 bis 200 nm aufweisen.

16. Lithiummetasilikat-Glaskeramik, die Lithiummetasilikat insbesondere als Hauptkristallphase und die Komponenten der Glaskeramik nach einem der Ansprüche 1 bis 13 enthält.

17. Glaskeramik nach einem der Ansprüche 1 bis 15 oder Lithiummetasilikat-Glaskeramik nach Anspruch 16, wobei die Glaskeramik und die Lithiummetasilikat-Glaskeramik in Form von einem Pulver, einem Granulat, einem Rohling oder einer dentalen Restauration vorliegen.

18. Verfahren zur Herstellung der Glaskeramik gemäß einem der Ansprüche 1 bis 15, bei dem ein Ausgangsglas, das die Komponenten der Glaskeramik enthält, oder die Lithiummetasilikat-Glaskeramik gemäß Anspruch 16 mindestens einer Wärmebehandlung im Bereich von 450 bis 1000°C unterzogen wird.

19. Verfahren nach Anspruch 18, bei dem

(a) das Ausgangsglas einer Wärmebehandlung bei einer Temperatur von 450 bis 600°C unterworfen wird, um Ausgangsglas mit Keimen zu bilden, und

(b) das Ausgangsglas mit Keimen einer Wärmebehandlung bei einer Temperatur von 700 bis 1000°C unterworfen wird, um die Lithiumdisilikat-Apatit-Glaskeramik zu bilden.

20. Verwendung der Lithiumdisilikat-Apatit-Glaskeramik gemäß einem der Ansprüche 1 bis 15 oder 17 oder der Lithiummetasilikat-Glaskeramik gemäß Anspruch 16 oder 17 als Dentalmaterial, insbesondere zur Beschichtung dentaler Restaurationen und bevorzugt zur Herstellung dentaler Restaurationen.

21. Verwendung zur Herstellung dentaler Restaurationen nach Anspruch 20, wobei der Lithiumdisilikat-Apatit-Glaskeramik oder der Lithiummetasilikat-Glaskeramik durch Verpressen oder maschinelle Bearbeitung die Form der gewünschten dentalen Restauration, insbesondere Brücke, Inlay, Onlay, Veneer, Abutment, Teilkrone, Krone oder Schale, gegeben wird.

**Claims**

1. Lithium disilicate-apatite glass ceramic, which comprises lithium disilicate as main crystal phase and apatite as further crystal phase, and which comprises divalent oxide selected from the group of CaO, SrO and mixtures thereof and transition metal oxide selected from the group of oxides of transition metals with an atomic number from 39 to 79 and mixtures thereof, wherein the molar ratio of divalent oxide to transition metal oxide is in the range of from 1.0 to 20.0, in particular 1.0 to 17.0 and preferably 1.5 to 16.5.

2. Glass ceramic according to claim 1, which comprises 52.0 to 75.0, in particular 54.0 to 73.0 wt.-% $SiO_2$.

3. Glass ceramic according to claim 1 or 2, which comprises 10.0 to 20.0, in particular 12.0 to 20.0 wt.-% $Li_2O$.

4. Glass ceramic according to any one of claims 1 to 3, which comprises 4.0 to 8.0 wt.-% $P_2O_5$.

5. Glass ceramic according to any one of claims 1 to 4, which comprises 2.0 to 9.0, in particular 3.0 to 8.0 wt.-% divalent oxide.

6. Glass ceramic according to any one of claims 1 to 5, which comprises 2.5 to 8.5, in particular 3.0 to 8.0 wt.-% CaO.

7. Glass ceramic according to any one of claims 1 to 6, which comprises 1.0 to 6.5, in particular 1.0 to 6.0 wt.-% SrO.

8. Glass ceramic according to any one of claims 1 to 7, which comprises 0.1 to 1.5, in particular 0.3 to 1.0 wt.-% F.

9. Glass ceramic according to any one of claims 1 to 8, which comprises 0 to 4.0, in particular 1.0 to 4.0 and preferably 1.5 to 4.0 wt.-% $Al_2O_3$.

10. Glass ceramic according to any one of claims 1 to 9, which comprises 0.5 to 8.5, in particular 1.0 to 8.0 and preferably 2.0 to 7.5 wt.-% transition metal oxide.

11. Glass ceramic according to any one of claims 1 to 10, in which the transition metal oxide is selected from the group of $La_2O_3$, $Y_2O_3$, $Er_2O_3$, $ZrO_2$, $CeO_2$, $Tb_4O_7$, $V_2O_5$, $Ta_2O_5$, $Nb_2O_5$ and mixtures thereof.

12. Glass ceramic according to any one of claims 1 to 11, in which the transition metal oxide is present
according to the formula $Me_2O_3$ in an amount of from 0 to 5.0, in particular 2.5 to 4.0 wt.-%.
according to the formula $MeO_2$ in an amount of from 0 to 6.5, in particular 1.0 to 6.0 wt.-%.
according to the formula $Me_4O_7$ in an amount of from 0 to 1.0, in particular 0.4 to 1.0 wt.-% and/or
according to the formula $Me_2O_5$ in an amount of from 0 to 5.0, in particular 0.1 to 4.0 wt.-%.

13. Glass ceramic according to any one of claims 1 to 12, which comprises monovalent oxide selected from the group of $Na_2O$, $K_2O$, $Rb_2O$, $Cs_2O$ and mixtures thereof in an amount of from 0 to 12.0, in particular 2.0 to 12.0 and preferably 3.0 to 11.5 wt.-%.

14. Glass ceramic according to any one of claims 1 to 13, which comprises fluoroapatite as apatite.

15. Glass ceramic according to any one of claims 1 to 14, in which the apatite crystal phase makes up 0.5 to 10, in particular 1 to 10 and preferably 2 to 8 wt.-% of the glass ceramic and/or the apatite crystals have an average size of from 5 to 500, in particular 10 to 300 and preferably 20 to 200 nm.

16. Lithium metasilicate glass ceramic, which comprises lithium metasilicate in particular as main crystal phase and the components of the glass ceramic according to any one of claims 1 to 13.

17. Glass ceramic according to any one of claims 1 to 15 or lithium metasilicate glass ceramic according to claim 16, wherein the glass ceramic and the lithium metasilicate glass ceramic are present in the form of a powder, a granulate, a blank or a dental restoration.

18. Process for the preparation of the glass ceramic according to any one of claims 1 to 15, wherein a starting glass comprising the components of the glass ceramic, or the lithium metasilicate glass ceramic according to claim 16 is subjected to at least one heat treatment in the range of from 450° to 1000°C.

19. Process according to claim 18, wherein

   (a) the starting glass is subjected to a heat treatment at a temperature of from 450 to 600°C in order to form starting glass with nuclei, and
   (b) the starting glass with nuclei is subjected to a heat treatment at a temperature of from 700 to 1000°C in order to form the lithium disilicate-apatite glass ceramic.

20. Use of the lithium disilicate-apatite glass ceramic according to any one of claims 1 to 15 or 17 or the lithium metasilicate glass ceramic according to claim 16 or 17 as dental material, in particular for coating dental restorations and preferably for the preparation of dental restorations.

21. Use for the preparation of dental restorations according to claim 20, wherein the lithium disilicate-apatite glass ceramic or the lithium metasilicate glass ceramic is given, by pressing or machining, the shape of the desired dental restoration, in particular bridge, inlay, onlay, veneer, abutment, partial crown, crown or facet.

## Revendications

1. Vitrocéramique de disilicate de lithium et d'apatite, qui contient du disilicate de lithium en tant que phase cristalline principale et de l'apatite en tant que phase cristalline supplémentaire, et qui contient un oxyde d'élément divalent, choisi dans l'ensemble formé par les oxydes CaO et SrO et leurs mélanges, et un oxyde de métal de transition, choisi dans l'ensemble formé par les oxydes des métaux de transition dont le numéro atomique vaut de 39 à 79 et leurs mélanges, étant entendu que le rapport molaire de l'oxyde d'élément divalent à l'oxyde de métal de transition se situe dans l'intervalle allant de 1,0 à 20,0, en particulier de 1,0 à 17,0, et de préférence de 1,5 à 16,5.

2. Vitrocéramique conforme à la revendication 1, qui contient de 52,0 à 75,0 % et en particulier de 54,0 à 73,0 % en poids de $SiO_2$.

3. Vitrocéramique conforme à la revendication 1 ou 2, qui contient de 10,0 à 20,0 % et en particulier de 12,0 à 20,0 % en poids de $Li_2O$.

4. Vitrocéramique conforme à l'une des revendications 1 à 3, qui contient de 4,0 à 8,0 % en poids de $P_2O_5$.

5. Vitrocéramique conforme à l'une des revendications 1 à 4, qui contient de 2,0 à 9,0 % et en particulier de 3,0 à 8,0 % en poids d'oxyde d'élément divalent.

6. Vitrocéramique conforme à l'une des revendications 1 à 5, qui contient de 2,5 à 8,5 % et en particulier de 3,0 à 8,0 % en poids de CaO.

7. Vitrocéramique conforme à l'une des revendications 1 à 6, qui contient de 1,0 à 6,5 % et en particulier de 1,0 à 6,0 % en poids de SrO.

8. Vitrocéramique conforme à l'une des revendications 1 à 7, qui contient de 0,1 à 1,5 % et en particulier de 0,3 à 1,0 % en poids de fluor.

**9.** Vitrocéramique conforme à l'une des revendications 1 à 8, qui contient de 0 à 4,0 %, en particulier de 1,0 à 4,0 % et de préférence de 1,5 à 4,0 % en poids de $Al_2O_3$.

**10.** Vitrocéramique conforme à l'une des revendications 1 à 9, qui contient de 0,5 à 8,5 %, en particulier de 1,0 à 8,0 % et de préférence de 2,0 à 7,5 % en poids d'oxyde de métal de transition.

**11.** Vitrocéramique conforme à l'une des revendications 1 à 10, dans laquelle l'oxyde de métal de transition est choisi dans l'ensemble formé par les suivants : $La_2O_3$, $Y_2O_3$, $Er_2O_3$, $ZrO_2$, $CeO_2$, $Tb_4O_7$, $V_2O_5$, $Ta_2O_5$, $Nb_2O_5$, et leurs mélanges.

**12.** Vitrocéramique conforme à l'une des revendications 1 à 11, dans laquelle un oxyde de métal de transition

- correspondant à une formule de type $Me_2O_3$ se trouve présent en une proportion de 0 à 5,0 % et en particulier de 2,5 à 4,0 % en poids,
- correspondant à une formule de type $MeO_2$ se trouve présent en une proportion de 0 à 6,5 % et en particulier de 1,0 à 6,0 % en poids,
- correspondant à une formule de type $Me_4O_7$ se trouve présent en une proportion de 0 à 1,0 % et en particulier de 0,4 à 1,0 % en poids,
- correspondant à une formule de type $Me_2O_5$ se trouve présent en une proportion de 0 à 5,0 % et en particulier de 0,1 à 4,0 % en poids.

**13.** Vitrocéramique conforme à l'une des revendications 1 à 12, qui contient un oxyde d'élément monovalent, choisi dans l'ensemble formé par $Na_2O$, $K_2O$, $Rb_2O$, $Cs_2O$ et leurs mélanges, en une proportion de 0 à 12,0 %, en particulier de 2,0 à 12,0 % et de préférence de 3,0 à 11,5 % en poids.

**14.** Vitrocéramique conforme à l'une des revendications 1 à 13, qui contient de la fluorapatite en guise d'apatite.

**15.** Vitrocéramique conforme à l'une des revendications 1 à 14, dans laquelle la phase cristalline d'apatite représente de 0,5 à 10 %, en particulier de 1 à 10 % et de préférence de 2 à 8 % du poids de la vitrocéramique et/ou les cristaux d'apatite présentent une taille moyenne de 5 à 500 nm, en particulier de 10 à 300 nm et de préférence de 20 à 200 nm.

**16.** Vitrocéramique de métasilicate de lithium, qui contient du métasilicate de lithium, en particulier en tant que phase cristalline principale, et les composants d'une vitrocéramique conforme à l'une des revendications 1 à 13.

**17.** Vitrocéramique conforme à l'une des revendications 1 à 15, ou vitrocéramique de métasilicate de lithium conforme à la revendication 16, lesquelles vitrocéramique et vitrocéramique de métasilicate de lithium se présentent sous la forme d'une poudre, d'un granulat, d'un matériau brut ou d'une pièce de restauration dentaire.

**18.** Procédé de préparation d'une vitrocéramique conforme à l'une des revendications 1 à 15, dans lequel on fait subir à un verre de départ qui contient les composants de la vitrocéramique, ou à une vitrocéramique de métasilicate de lithium conforme à la revendication 16, au moins un traitement thermique dans le domaine de 450 à 1000 °C.

**19.** Procédé conforme à la revendication 18, dans lequel

a) on soumet le verre de départ à un traitement thermique à une température de 450 à 600 °C, pour en faire un verre de départ comportant des germes,
b) et l'on soumet ce verre de départ comportant des germes à un traitement thermique à une température de 700 à 1000 °C, pour en faire une vitrocéramique de disilicate de lithium et d'apatite.

**20.** Utilisation d'une vitrocéramique de disilicate de lithium et d'apatite, conforme à l'une des revendications 1 à 15 et 17, ou d'une vitrocéramique de métasilicate de lithium conforme à la revendication 16 ou 17, en tant que matériau dentaire, en particulier pour le revêtement de restaurations dentaires et de préférence pour la fabrication de restaurations dentaires.

**21.** Utilisation pour fabrication de restaurations dentaires, conforme à la revendication 20, dans laquelle on donne à la vitrocéramique de disilicate de lithium et d'apatite ou à la vitrocéramique de métasilicate de lithium, par pressage ou par façonnage à la machine, la forme de la restauration dentaire voulue, en particulier bridge, incrustation in-lay ou on-lay, facette veneer, pilier, couronne partielle, couronne ou coquille.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013164256 A1
- US 6455451 B1
- EP 1505041 A1
- EP 231773 A

**In der Beschreibung aufgeführte Nicht-Patentliteratur**

- **M. PALOU et al.** *Cent. Eur. J. Chem,* 2009, vol. 7 (2), 228-233
- **S.C. MOJUMDAR et al.** *Journal of Thermal Analysis and Calorimetry,* 2004, vol. 78 (1), 73-82
- *Central European Journal of Chemistry,* 2009, vol. 7 (2), 228-233